# FASCICULE DE BREVET EUROPEEN

(11) **EP 0 612 507 B1**
(45) Date de publication et mention de la délivrance du brevet: **09.12.1998**
(21) Numéro de dépôt: 94420067.4
(22) Date de dépôt: 22.02.1994
(51) Int. Cl.: A61B 17/58

(54) **Fixateur pour les ostéosynthèses du rachis lombo-sacré**
Fixiermittel für die Osteosynthese der lumbosakralen Wirbelsäule
Fixator for osteosynthesis of the lumbosacral spine

(30) Priorité: 24.02.1993 FR 9302357; 09.02.1994 FR 9401661
(43) Date de publication de la demande: 31.08.1994
(73) Titulaire: SOCIETE DE FABRICATION DE MATERIEL ORTHOPEDIQUE SOFAMOR, 93290 Tremblay-en-France (FR)
(72) Inventeur: Fournet-Fayard, Jacques, F-26000 Valence (FR); Garin, Christophe, F-69006 Lyon (FR); Galland, Olivier, F-38240 Meylan (FR); Lucet, Alain, F-21000 Dijon (FR)
(74) Mandataire: Polus, Camille

(56) Documents cités:
- EP-A- 0 140 790
- EP-A- 0 441 729
- EP-A- 0 487 895
- FR-A- 2 289 164

## Description

La présente invention a trait à un fixateur pour des ostéosynthèses lombo-sacrées permettant le maintien et l'assemblage des étages vertébraux endommagés de cette partie du rachis. Ce fixateur comprend les moyens énumérés à la partie préambule de la revendication 1.

On connaît des fixateurs de ce genre qui comprennent généralement deux ensembles sensiblement parallèles comportant chacun :
- une tige cylindrique dont la longueur est fonction de la hauteur et du nombre des étages vertébraux à réunir ;
- et plusieurs ensembles composés chacun d'une bague, d'un écrou et d'une vis pédiculaire permettant la fixation des tiges dans les pédicules de chaque vertèbre.

De tels fixateurs nécessitent l'alignement des vis pédiculaires dans le plan sagittal et frontal. En effet, si l'alignement n'est pas respecté, la réunion et la connexion par la tige est impossible, à moins de déformer cette dernière pour récupérer le décalage angulaire.

De plus, les fixateurs considérés entraînent des risques de dégénérescence ultérieure du disque qui se trouve immédiatement sus-jacent à l'ensemble des vertèbres instrumentées. En effet, le disque reçoit après l'ostéosynthèse lombo-sacrée la totalité des contraintes qui, en l'état normal, se trouvent réparties sur plusieurs étages.

C'est à ces inconvénients qu'entend plus spécialement remédier la présente invention.

Le document EP-A-0 140 790 (D1) décrit un appareil de corrections rachidiennes, essentiellement de scolioses, de la partie déformée du rachis d'un enfant en cours de croissance, après quoi l'appareil est retiré. A cet effet cet appareil est constitué d'une structure élastique flexible de rappel, formée d'une ou plusieurs tiges flexibles, fixées à une vertèbre à une extrémité et coulissant librement dans des brides de retenue solidaires des vertèbres.

Le document EP-A-0 441 729 (D2) enseigne une tige vertébrale et une vis pédiculaire équipée d'une bague entourant la tige à l'intérieur du corps de la vis et permettant d'orienter la tige dans diverses directions afin de la bloquer dans une position angulaire désirée.

Ces deux dispositifs antérieurs sont inaptes à fournir une contribution à la résolution du problème ci-dessus, car ils sont sans rapport avec celui-ci.

Le fixateur pour ostéosynthèse du rachis lombo-sacré suivant la présente invention est conforme à la partie caractérisante de la revendication 1.

Le dessin annexé, donné à titre d'exemple, permettra de mieux comprendre l'invention, les caractéristiques qu'elle présente et les avantages qu'elle est susceptible de procurer :
Fig. 1 est une vue en élévation illustrant le fixateur pour ostéosynthèse du rachis lombo-sacré suivant l'invention après sa mise en place.
Fig. 2 et 3 sont des vues en perspective d'un moyen de guidage qui est destiné à recevoir la seconde partie de la tige de liaison du fixateur suivant la présente invention.
Fig.4 est une vue en coupe partielle et élévation illustrant le guidage de la seconde partie de la tige de liaison du fixateur.
Fig.5 et 6 sont des vues mi-coupe mi-élévation montrant des variantes des moyens de guidage de la seconde partie de la tige de liaison.

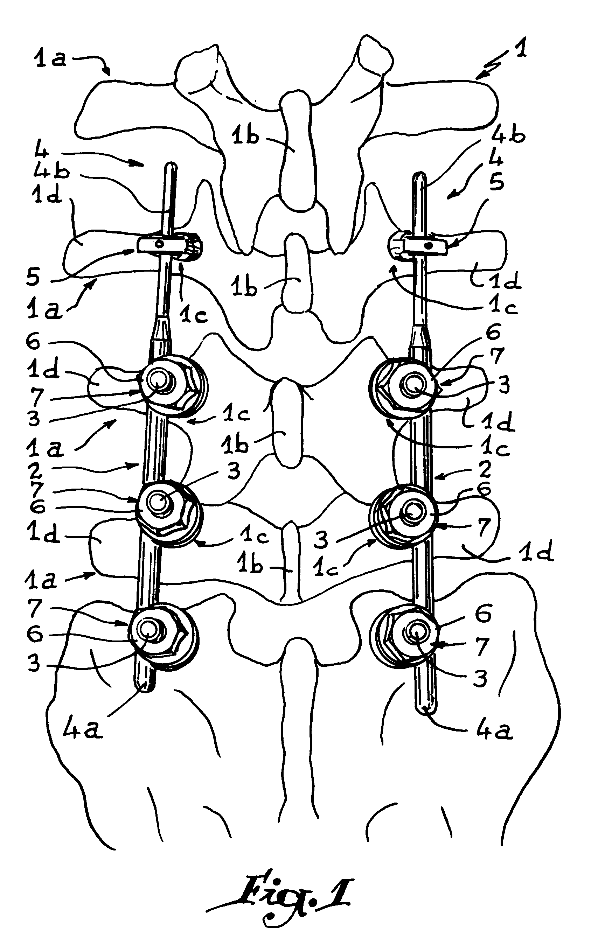

On a représenté en fig. 1 la partie basse d'une colonne vertébrale 1 dont les vertèbres 1a sont réunies les unes aux autres par un fixateur 2 afin de réaliser une ostéosynthèse lombo-sacrée, c'est-à-dire une soli-darisation des étages vertébraux endommagés au niveau considéré.

Le fixateur 2 comprend deux ensembles sensiblement parallèles qui sont placés de part et d'autre des apophyses épineuses 1b et immobilisés sur les pédicules 1c.

Le fixateur 2 est constitué pour chaque ensemble d'un certain nombre de vis pédiculaires 3 qui sont ancrées dans les pédicules 1c de chaque vertèbre 1a et qui comportent des moyens de serrage 7 connus en soi pour le maintien d'une tige 4 de liaison. La tige de liaison 4 permet de réunir chaque vis pédiculaire 3 préalablement ancrée dans les vertèbres 1a.

La tige cylindrique de liaison 4 est généralement prévue cintrée, soit dans le sens convexe, soit dans le sens concave, suivant la morphologie du patient et la correction à obtenir. La tige 4 est prévue d'une longueur suffisante pour relier un certain nombre de vertèbres 1a dont les disques sont endommagés.

La tige 4 comporte une première partie 4a qui est prévue pour coopérer avec les moyens de serrage 7 des vis pédiculaires 3 lors du montage du fixateur 2.

La première partie 4a de la tige 4 se prolonge par une seconde partie 4b de diamètre inférieur. Cette seconde partie 4b peut être prévue par exemple pour former une tige 4 à segmentation régulière, c'està-dire que la partie 4a est encadrée par deux parties 4b et inversement une partie 4b est encadrée par deux parties 4a. La seconde partie 4b est destinée à coopérer avec des moyens de guidage 5 dont on décrira mieux plus loin la fonction. Les moyens de guidage 5 sont montés sur chaque apophyse transversale 1d de la vertèbre sus-jacente 1a à la dernière vertèbre instrumentée, de manière à créer une transition semi-rigide entre la vertèbre normale 1a et celle retenue par le fixateur 2 pour éviter la création du syndrome de "néo-charnière".

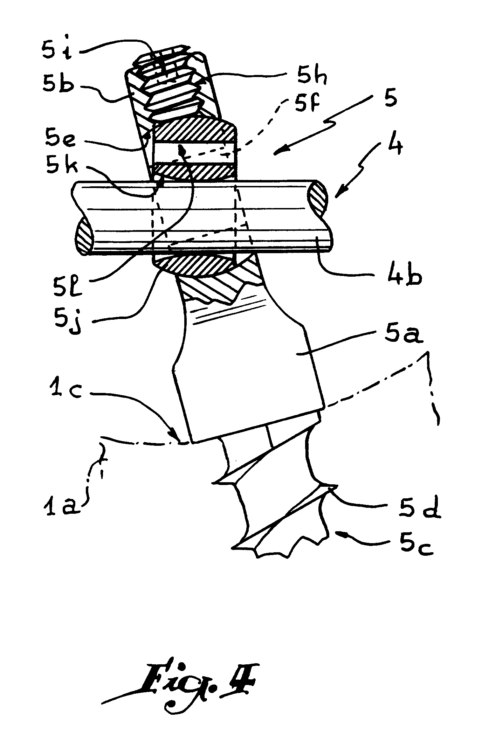

On a représenté en fig. 2 à 4 les moyens de guidage 5 de la seconde partie 4b de la tige de liaison 4 en vue de réaliser un soulagement des efforts qui sont supportés par le disque de la vertèbre 1a se trouvant juste au-dessus de la dernière vertèbre 1a instrumentée au moyen du fixateur 2.

Les moyens de guidage 5 sont constitués d'une vis 5a dont la tête 5b présente la forme d'un anneau. La vis 5a comporte au-dessous de sa tête 5b une tige 5c pourvue d'un filet auto-taraudant 5d permettant son ancrage dans le pédicule 1c de la vertèbre 1a qui se trouve au-dessus de la dernière vertèbre instrumentée.

La tête 5b est percée suivant un axe transversal par rapport à celui de la tige 5c d'un alésage 5e présentant un profil interne en portion de sphère. De part et d'autre de l'alésage 5e sont prévues deux encoches latérales débouchantes 5f et 5g dont le diamètre interne est supérieur à celui dudit alésage. Dans la partie supérieure de la tête 5b et suivant un axe vertical est percé un trou taraudé 5h débouchant à l'intérieur de l'alésage 5e et à l'intérieur duquel est vissée une vis 5i.

A l'intérieur de l'alésage 5e est introduit un disque 5j afin de constituer une liaison du genre rotule. Le disque 5j est percé d'un premier trou 5k qui est prévu décalé angulairement par rapport aux axes principaux dudit disque. Le trou 5k présente un profil interne bombé convexe dont le diamètre est légèrement supérieur à celui de la partie 4b de la tige 4 afin que cette dernière puisse coulisser librement à l'intérieur dudit disque 5j, comme on le verra mieux plus loin. De plus, le profil bombé du trou 5k permet de Limiter la surface en contact avec la partie 4b pour réduire les efforts de frottement entre celle-ci et l'intérieur du trou 5k. A proximité du trou 5k est percé un second trou 5l de diamètre inférieur et permettant au chirurgien d'orienter le disque 5j à l'intérieur de l'alésage 5e de la vis 5a. Le disque 5j présente un profil externe en portion de sphère de même rayon que celui interne de l'alésage 5e, de manière à constituer une liaison du type rotule permettant audit disque 5j de se déplacer librement suivant différentes orientations angulaires à l'intérieur dudit alésage 5e.

Les tiges 4 et plus particulièrement leurs parties 4a coopèrent avec les vis pédiculaires 3 préalablement ancrées dans les pédicules 1c de chaque vertèbre 1a à soulager, tandis que leurs parties 4b coopèrent avec les vis anneaux 5 qui sont montées dans chaque pédicule 1c de la vertèbre sous-jacente 1a à la dernière vertèbre instrumentée, de manière à créer une position semi-rigide entre la vertèbre normale 1a et celle retenue par le fixateur 2.

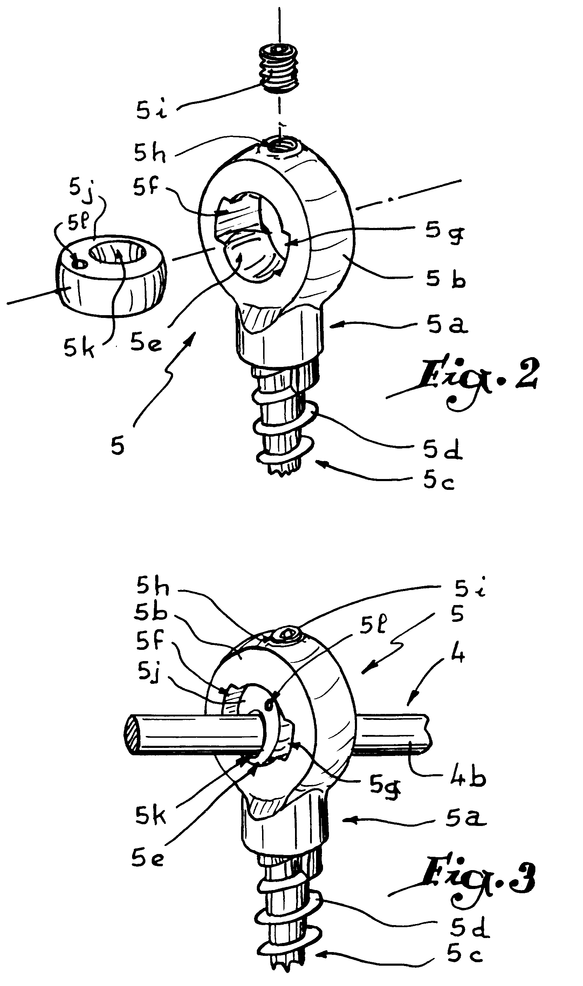

Avant la mise en place des vis-anneaux 5, le chirurgien introduit à l'intérieur de l'alésage 5e le disque 5j de manière qu'il reste libre en rotation angulaire. Lors de l'introduction de la tige 4 et plus particulièrement de la partie 4b à l'intérieur du trou 5k du disque 5j, le chirurgien déplace angulairement ce dernier au moyen d'un outil approprié qui coopère avec le trou 5l afin de déplacer ledit disque dans la bonne direction comme représenté en fig. 3. Ensuite, le chirurgien immobilise le disque 5j à l'intérieur de l'alésage 5e par l'intermédiaire de la vis 5i.

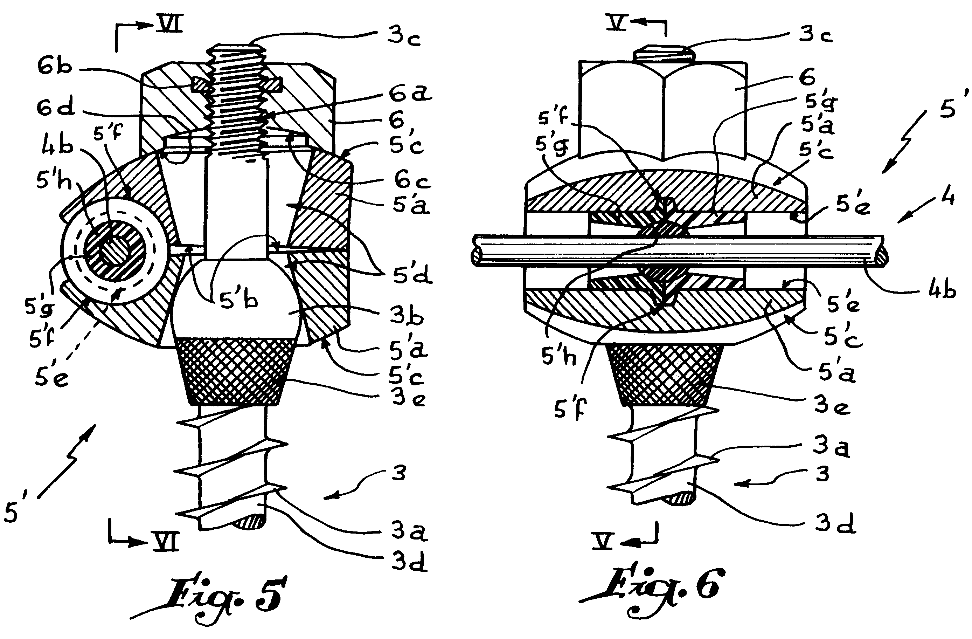

On a représenté en fig. 5 et 6 une variante des moyens de guidage 5 référencés 5' de la seconde partie 4b de la tige de liaison 4.

Les moyens de guidage 5' sont montés sur des vis pédiculaires 3 identiques à celles permettant la mise en place de la partie 4a de la tige 4 et recevant les moyens de serrage 7, comme décrit en fig. 1.

Chaque vis pédiculaire 3 comprend un filet auto-taraudant 3a et une tête sphérique 3b dont la partie supérieure se prolonge par une tige filetée 3c co-axiale au filet 3a.

Le filet auto-taraudant 3a comporte une âme 3d à profil conique de manière à assurer une meilleure stabilité de la partie supérieure de la vis. La base la plus large du profil conique de l'âme 3d est prévue du côté de la tête sphérique 3b de la vis pédiculaire 3.

La tête sphérique 3b se prolonge du côté du filet auto-taraudant 3a par une partie conique 3e dont l'état de surface est rugueux pour permettre un ancrage biologique, progressif et durable pour la reprise de l'os autour de la tête.

La tige filetée 3c est prévue de longueur variable suivant le point d'ancrage de la vis pédiculaire 3. De plus, la tige filetée 3c peut-être pourvue de zones de rupture afin qu'elle soit sécable en fonction de la hauteur utilisée.

Les moyens de guidage 5' sont constitués par deux rondelles 5'a identiques et symétriques pouvant former réciproquement soit la partie inférieure, soit la partie supérieure desdits moyens de guidage. Chacune des rondelles 5'a présente une surface d'appui plane 5'b et un profil extérieur sphérique 5'c. Chaque rondelle 5'a est percée d'un alésage débouchant 5'd qui est décalé latéralement par rapport à l'axe vertical desdites rondelles. L'alésage 5'd présente un profil conique dont la base la plus large est tournée du côté du profil sphérique 5'c des rondelles 5'a.

Des encoches 5'e sont ménagées perpendiculairement à l'axe de l'alésage tronconique 5'd et comportent en leur milieu une rainure en V 5'f. Celle-ci permet la mise en place d'une bague en nylon 5'g comprenant en son milieu une rotule 5'h qui est percée d'un alésage traversant pour le coulissement de la seconde partie 4b de la tige 4. La rainure 5'f peut être prévue d'un profil quelconque de manière qu'elle retienne axialement la bague 5'g dans les encoches 5'e. Les rondelles 5'a sont immobilisées sur la vis pédiculaires 3 par l'intermédiaire d'un écrou 6 comportant un trou taraudé 6a dans lequel est prévu une rondelle frein 6b. Chaque écrou se visse sur la tige filetée 3c des vis pédiculaires 3.

Chaque écrou 6 comporte un chambrage 6c qui permet de délimiter sur la face d'appui dudit écrou une surface sphérique 6d. La surface sphérique 6d est prévue d'un rayon correspondant à celui du profil 5'c des rondelles 5'a.

On note que les moyens de guidage 5 et 5' créent une transition semi-rigide entre la vertèbre normale 1a et celle retenue par le fixateur 2 pour éviter la création du syndrome de "néo-charnières".

## Revendications

1. Fixateur pour ostéosynthèse du rachis lombo-sacré comprenant deux ensembles sensiblement parallèles pour la fusion des étages vertébraux endommagés d'une colonne vertébrale, chaque ensemble comportant des vis pédiculaires (3), une tige de liaison (4) entre les vis et des moyens de serrage (7) pour le maintien de la tige, caractérisé en ce que la tige de liaison (4) présente une première partie (4a) qui coopère avec les moyens de serrage (7) prévus sur les vis pédiculaires (3), une seconde partie (4b) de diamètre inférieur à celui de la première, et en ce que le fixateur comporte des moyens de guidage (5, 5') adaptés pour que ladite seconde partie de la tige de liaison coulisse librement de dans, afin de permettre un soulagement des efforts qui sont supportés par le disque de la vertèbre (1a) se trouvant juste au-dessus de la dernière vertèbre (1a) instrumentée au moyen du fixateur (2).

2. Fixateur suivant la revendication 1, caractérisé en ce que les moyens de guidage (5) consistent en une vis-anneau (5a) comportant une tête (5b) percée d'un alésage (5e) pourvu de deux encoches latérales (5f) pour la mise en place d'un disque (5j) dans lequel est percé un trou bombé (5k) pour le passage de la seconde partie (4b) de la tige (4), tandis que le disque (5j) est immobilisé en rotation à l'intérieur de l'alésage (5e) par une vis de pression (5i).

3. Fixateur suivant la revendication 2, caractérisé en ce que l'alésage (5e) présente un profil interne en portion de sphère.

4. Fixateur suivant la revendication 2, caractérisé en ce que le disque (5j) présente un profil externe identique à celui de l'alésage (5e) pour former une liaison du type rotule.

5. Fixateur suivant la revendication 2, caractérisé en ce que le disque (5j) est percé d'un premier trou (5k) à profil bombé et d'un second trou (5l) de diamètre inférieur.

6. Fixateur suivant la revendication 2, caractérisé en ce que la visanneau (5) comporte une tige (5c) pourvue d'un filet auto-taraudant (5d).

7. Fixateur suivant la revendication 1, caractérisé en ce que les moyens de guidage (5') sont constitués par des rondelles symétriques (5'a) dont chacune est pourvue d'un alésage débouchant (5'd) à profil conique et par au moins une encoche (5'e) qui est ménagée perpendiculairement audit alésage et à l'intérieur de laquelle est prévue en son milieu une rainure (5'f) permettant la retenue d'une bague en Nylon (5'g) qui comprend une rotule (5'h) dans laquelle est percé un alésage débouchant pour le coulissement de la seconde partie (4b) de la tige de liaison (4).

8. Fixateur suivant la revendication 7, caractérisé en ce que les rondelles (5'a) sont immobilisées sur une vis pédiculaire (3) au moyen d'un écrou (6).

9. Fixateur suivant la revendication 7, caractérisé en ce que la rainure (5'f) présente un profil en forme de V.

10. Fixateur suivant la revendication 7, caractérisé en ce que la vis pédiculaire (3) comprend un filet auto-taraudant (3a) et une tête sphérique (3b) qui est solidaire dans sa partie supérieure et co-axialement audit filet d'une tige filetée (3c) de hauteur variable, tandis que la tête sphérique (3b) présente dans sa partie intérieure un profil conique (3e) dont l'état de surface est rugueux pour permettre la reprise osseuse.

## Claims

1. Fixing member for osteosynthesis of the sacrolumbar spine, comprising two substantially parallel assemblies for fusing the damaged vertebral stages of a spinal column, each assembly comprising grub screws (3), a connecting rod (4) between the screws and gripping means (7) for holding the rod, characterised in that the connecting rod (4) has a first part (4*a*) which co-operates with the gripping means (7) provided on the grub screws (3), a second part (4*b*) smaller in diameter than the first, and in that the fixing member comprises guide means (5, 5') adapted so that said second part of the connecting rod slides freely therein, so as to allow the releif of the forces which are borne by the disc of the vertebra (1*a*) located just above the last vertebra (1*a*) secured by the fixing member (2).

2. Fixing member according to claim 1, characterised in that the guide means (5) consists of a ring screw (5*a*) comprising a head (5*b*) drilled with a bore (5*e*) having two lateral notches (5*f*) for the positioning of a disc (5*j*) in which is drilled a dished hole (5*k*) for accommodating the second part (4*b*) of the rod (4), whilst the disc (5*j*) is secured against rotation inside the bore (5*e*) by a pressure screw (5*i*).

3. Fixing member according to claim 2, characterised in that the bore (5*e*) has an internal profile shaped like a portion of a sphere.

4. Fixing member according to claim 2, characterised in that the disc (5*j*) has an external profile identical to that of the bore (5*e*) to form a ball-and-socket joint.

5. Fixing member according to claim 2, characterised in that the disc (5*j*) is drilled with a first hole (5*k*) having a dished profile and a second hole (5*l*) which is smaller in diameter.

6. Fixing member according to claim 2, characterised in that the ring screw (5) comprises a rod (5*c*) provided with a self-tapping thread (5*d*).

7. Fixing member according to claim 1, characterised in that the guide means (5') consist of symmetrical discs (5'*a*) each of which is provided with a through-bore (5'*d*) which is conical in profile and of at least one notch (5'*e*) which is formed perpendicularly to said bore and inside which is provided, in the centre, a groove (5'*f*) for retaining a Nylon ring (5'*g*) which comprises a ball-and-socket (5'*h*) in which is drilled a through-hole for the sliding of the second part (4*b*) of the connecting rod (4).

8. Fixing member according to claim 7, characterised in that the discs (5'*a*) are secured to a grub screw (3) by a nut (6).

9. Fixing member according to claim 7, characterised in that the groove (5'*f*) has a V-shaped profile.

10. Fixing member according to claim 7, characterised in that the grub screw (3) comprises a self-tapping thread (3*a*) and a spherical head (3*b*) which is fixedly connected at its upper end, coaxially with said thread, to a threaded rod (3*c*) of variable height, whereas the spherical head (3*b*) has, in its inner part, a conical profile (3*e*) the surface of which is rough so as to allow the bone to take.

## Patentansprüche

1. Fixiereinrichtung für die Osteosynthese der lumbosakralen Wirbelsäule, bestehend aus zwei im wesentlichen parallelen Einheiten für die Fusion der beschädigten Wirbeletagen einer Wirbelsäule, wobei jede Einheit Schrauben (Pedikularschrauben) (3), eine Verbindungsstange (4) zwischen den Schrauben und Klemmeinrichtungen (7) für den Halt der Stange aufweist, dadurch gekennzeichnet, daß die Verbindungsstange (4) einen ersten Teil (4a), der mit den auf den Schrauben (3) vorgesehenen Klemmeinrichtungen (7) zusammenwirkt, und einen zweiten Teil (4b) mit kleinerem Durchmesser als der erste Teil aufweist und daß die Fixiereinrichtung Führungseinrichtungen (5, 5') aufweist, die so ausgelegt sind, daß der zweite Teil der Verbindungsstange darin frei gleitet, um den Diskus des Wirbels (1a), der sich unmittelbar über dem letzten mit der Fixiereinrichtung (2) bestückten Wirbel befindet, von den auf ihn ausgeübten Kräften zu entlasten.

2. Fixiereinrichtung nach Anspruch 1, dadurch gekennzeichnet, daß die Führungseinrichtungen (5) aus einer Ringschraube (5a) bestehen, die einen Kopf (5b) aufweist, der mit einer Bohrung (5e) versehen ist, die zwei seitliche Ausschnitte (5f) zum Einsetzen einer Scheibe (5j) aufweist, in der eine gewölbte Bohrung (5k) für den Durchgang des zweiten Teils (4b) der Stange (4) vorgesehen ist, während die Scheibe (5j) im Inneren der Bohrung (5e) durch eine Druckschraube (5i) bezüglich Drehung blockiert ist.

3. Fixiereinrichtung nach Anspruch 2, dadurch gekennzeichnet, daß die Bohrung (5e) ein Innenprofil in Form eines Kugelteils aufweist.

4. Fixiereinrichtung nach Anspruch 2, dadurch gekennzeichnet, daß die Scheibe (5j) ein äußeres Profil aufweist, das mit dem der Bohrung (5e) identisch ist, um eine Kugelgelenkverbindung zu bilden.

5. Fixiereinrichtung nach Anspruch 2, dadurch gekennzeichnet, daß die Scheibe (5j) mit einer ersten Bohrung (5k) mit gewölbtem Profil und mit einer zweiten Bohrung (5l) mit kleinerem Durchmesser versehen ist.

6. Fixiereinrichtung nach Anspruch 2, dadurch gekennzeichnet, daß die Ringschraube (5) einen mit einem selbstschneidenden Gewinde (5d) versehenen Schaft (5c) aufweist.

7. Fixiereinrichtung nach Anspruch 1, dadurch gekennzeichnet, daß die Führungseinrichtungen (5') aus symmetrischen Ringen (5'a) bestehen, deren jeder mit einer ausmündenden Bohrung (5'd) mit konischem Profil versehen ist, und aus mindestens einer Aussparung (5'e), die senkrecht zu der Bohrung angeordnet ist und in deren Innerem in der Mitte eine Nut (5'f) vorgesehen ist, die die Zurückhaltung eines Ringes (5'g) aus Nylon gestattet, der einen Kugelgelenkkopf (5'h) aufweist, in dem eine ausmündende Bohrung vorgesehen ist, in der der zweite Teil (4b) der Verbindungsstange (4) gleiten kann.

8. Fixiereinrichtung nach Anspruch 7, dadurch gekennzeichnet, daß die Ringe (5'a) auf einer Schraube (3) mit Hilfe einer Mutter (6) blockiert sind.

9. Fixiereinrichtung nach Anspruch 7, dadurch gekennzeichnet, daß die Nut (5'f) ein V-förmiges Profil aufweist.

10. Fixiereinrichtung nach Anspruch 7, dadurch gekennzeichnet, daß die Pedikulumschraube (3) ein selbstschneidendes Gewinde (3a) und einen kugelförmigen Kopf (3b) aufweist, der in seinem oberen Teil koaxial zu diesem Gewinde mit einer Gewindestange (3c) veränderlicher Höhe fest verbunden ist, während der kugelförmige Kopf (3b) in seinem unteren Teil ein konisches Profil (3e) aufweist, das einen aufgerauhten Oberflächenzustand besitzt, um das Nachwachsen des Knochens zu gewährleisten.
